# EUROPÄISCHE PATENTSCHRIFT

(11) **EP 0 879 813 B1**
(45) Veröffentlichungstag und Bekanntmachung des Hinweises auf die Patenterteilung: **20.03.2002**
(21) Anmeldenummer: 98106514.7
(22) Anmeldetag: 08.04.1998
(51) Int. Cl.: C07C 61/04, C07C 51/09

(54) **Verfahren zur Herstellung von Cyclopropancarbonsäuren**
Process of preparation of cyclopropane carboxylic acids
Procédé de préparation d'acides cyclopropane carboxyliques

(30) Priorität: 22.05.1997 DE 19721426
(43) Veröffentlichungstag der Anmeldung: 25.11.1998
(73) Patentinhaber: Degussa AG, 40474 Düsseldorf (DE)
(72) Erfinder: Kaufhold, Manfred, Dr., 45770 Marl (DE)

(56) Entgegenhaltungen:
- FRIEDHELM KORTE: "Methodicum Chimicum" 1975 , GEORG THIEME VERLAG , STUTTGART XP002075408 * Seite 563-564, insb. vgl. Seite 564, Absatz 3 *

## Beschreibung

Die Erfindung betrifft ein neues Verfahren zur Herstellung von Cyclopropancarbonsäuren durch Acidolyse der entsprechenden Ester nach folgendem Schema:

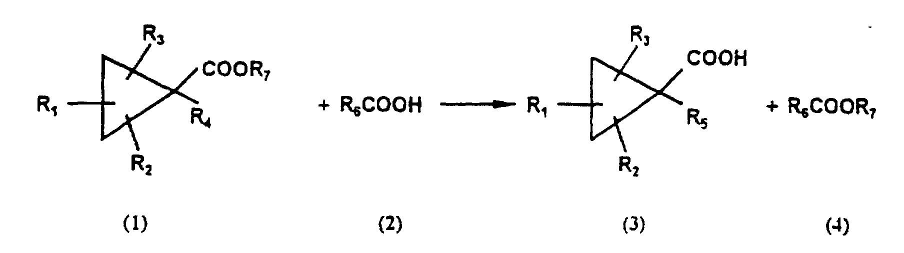

Dabei ist
- R₁, R₂, R₃ =: H, C₁- bis C₄-Alkyl, Phenyl,
- R₄ =: H, COOR₇,
- R₅ =: H, COOR₇, COOH,
- R₆ =: H, CH₃, C₂H₅, C₃H₇,
- R₇ =: CH₃, C₂H₅

Cyclopropancarbonsäuren sind wichtige Zwischenprodukte bei der Herstellung von asymmetrischen Chemikalien, Pharmaprodukten und Pflanzenschutzmitteln

Die Cyclopropancarbonsäureester (1) werden nach bekannten Methoden hergestellt Der Cyclopropancarbonsauremethylester wird beispielsweise durch Umsetzung von 4-Chlorbuttersäuremethylester mit Natriummethylat nach EP-A-0 577 949 synthetisiert.

Aus der Literatur sind auch Synthesen von Cyclopropancarbonsäuren (3) bekannt. So wird in Org. Synth Coll Vol. 3 (1955), 221, die alkalische Verseifung des entsprechenden Nitrils beschrieben. Bei diesem Verfahren fallen Ammoniak und Alkalisalze als Abfallprodukte in stöchiometrischen Mengen an, die entsorgt werden mussen

Bei der alkalischen Verseifung von Cyclopropancarbonsäureestern hat man ebenfalls das Problem des Salzanfalls in stöchiometrischen Mengen.

Bei der Acidolyse ist der Salzanfall dagegen geringer, da dieses Verfahren katalytisch durchgeführt wird. Eingehende Beschreibungen der Acidolyse befinden sich in Lehrbüchern, so z.B. im Organikum, 20. Auflage, Johann Ambrosius Barth Verlag (1996), 459. Als Katalysator wird dabei Schwefelsäure und als Carbonsäure Ameisensäure empfohlen.

Im Fall der Cyclopropancarbonsäuren sind bei einer derartigen Acidolyse relativ große Mengen an Schwefelsäure erforderlich. Dabei muß diese Säure nach der Reaktion beispielsweise mit Natronlauge neutralisiert werden, da die empfindlichen Cyclocarbonsäuren sonst bei der destillativen Aufarbeitung zersetzt werden. Daher fallen also auch bei dieser Arbeitsweise Salzmengen an, die abgetrennt und entsorgt werden müssen.

Friedhelm Korte, Methodicum Chimicum, Georg Thieme Verlag, Stuttgart, 1975, Seiten 563 und 564, beschreibt ein Verfahren zur Herstellung von Säuren aus den entsprechenden Estern durch Umesterung. Dabei wird jedoch über verfahrenstechnische Probleme bei der Aufarbeitung und den Verbleib des Katalysators nichts berichtet.

Allen bekannten Verfahren zur Herstellung von Cyclopropancarbonsäuren ist also gemeinsam, daß sie einen hohen Verbrauch an Chemikalien haben, was zu Problemen bei der Abfallbeseitigung führt.

Aufgabe der vorliegenden Erfindung war es, Cyclopropancarbonsäuren herzustellen und dabei die genannten Nachteile zu vermeiden.

Diese Aufgabe wird überraschend dadurch gelöst, daß man als Katalysator bei der Acidolyse eine Alkylbenzolsulfonsäure der Formel 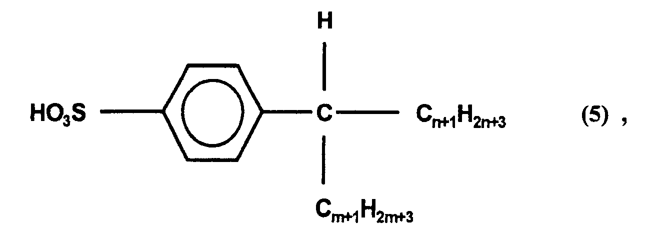 wobei m + n = 5 bis 15, vorzugsweise 7 bis 10, ist, einsetzt.

Für die Umsetzung ist kein Lösungsmittel erforderlich, man kann aber, wenn es sich z. B. um einen Feststoff handelt, Lösungsmittel mit einem geeigneten Siedepunkt zusetzen. Wichtig ist, daß der entstehende Ester (4) leicht durch Destillation aus dem Reaktionsgemisch abgetrennt werden kann, um auf diese Weise das Gleichgewicht schnell zur gewünschten Seite zu verschieben. Man setzt daher nur die Methyl- und Ethylester ein, wobei die Methylester vorzugsweise verwendet werden.

Außerdem kommen Carbonsäuren mit niedrigen Siedepunkten, wie Ameisen-, Essig-, Propion- und Buttersäure, zum Einsatz, wobei Ameisensäure bevorzugt wird. Aus wirtschaftlichen Gründen wird die Carbonsäure (2) im Überschuß zum Ester (1) eingesetzt.

Die als Katalysator eingesetzten Alkylbenzolsulfonsäuren weisen in 4-Stellung einen geradkettigen oder verzweigten Alkylrest auf, der vorzugsweise 10 bis 13 C-Atome enthält. Beispiele dafür sind eine Tetrapropyl-, n-Decyl-, n-Undecyl-, n-Dodecyl- und n-Tridecyl-Seitenkette. Aus wirtschaftlichen Gründen werden ganz besonders bevorzugt solche Alkylbenzolsulfonsäuren eingesetzt, die aus üblichen, technisch leicht herstellbaren und leicht verfügbaren Gemischen bestehen.

Der Katalysator wird in relativ großen Mengen von vorzugsweise 5 bis 50 Gew.-%, bezogen auf den Ester, zugegeben. Dabei werden Mengen von 7 bis 15 Gew.-% ganz besonders bevorzugt eingesetzt.

In einer besonders bevorzugten Ausführungsform der Erfindung werden die Reaktionsprodukte nach der Umsetzung abdestilliert, worauf das flüssige Sumpfprodukt als Katalysator für die nächste Reaktion wieder eingesetzt wird.

Diese Verfahrensweise ermöglicht der stabile Katalysator. Denn trotz der relativ großen Einsatzmengen an Alkylbenzolsulfonsäure findet weder eine Zersetzung zu Leichtsiedern, noch zu nicht destillierbaren Hochsiedern, die von der Alkylbenzolsulfonsäure nicht abtrennbar wären, in nennenswertem Umfange statt. Der Katalysator kann daher sehr oft zurückgeführt werden. Eine Entsorgung des Katalysators ist nicht erforderlich.

Das Verfahren wird dadurch technisch leicht handhabbar und wirtschaftlich. Durch Verwendung der hohen Katalysatorkonzentration läuft die Reaktion auch schnell und vollständig ab.

Diese Effekte waren nicht vorhersehbar.

Die praktische Durchführung des Verfahrens erfolgt z B auf folgende Weise: Der Cyclopropancarbonsäureester (1) und die Carbonsäure (2) werden vorgelegt, wobei das Molverhaltnis 1. 1 bis 1 : 20, vorzugsweise 1 : 1,1 bis 1 10 und besonders bevorzugt 1 1,1 bis 1 3 betragt Dann wird der Katalysator zugegeben und das Gemisch unter Ruhren erwärmt. Die Reaktion findet im allgemeinen bei 90 bis 150 °C statt. Dabei wird der Ester (4) abdestilliert, wobei auch leichtes Vakuum im Bereich von 500 bis 1 000 h Pa angelegt werden kann. Bei der Destillation kann auch eine Kolonne angewandt werden, um eine Destillation des Esters (1) zu vermeiden Wenn die Reaktion beendet ist, steigt die Kopftemperatur bei der Destillation an

Dann wird das Reaktionsgemisch vom Katalysator abdestilliert (abgetoppt) und der flüssige Destillationsruckstand in die nächste Umsetzung gegeben Das abdestillierte Reaktionsgemisch wird nach üblichen Methoden aufgearbeitet, beispielsweise also destillativ im Falle der flüssigen Cyclopropancarbonsaure oder durch Filtration und Umkristallisation im Falle von festen Carbonsäuren.

Das Verfahren kann auch kontinuierlich durchgeführt werden Dann wird z. B. Ameisensäure, Ester und Katalysator vorgelegt, ein Gemisch von Ameisensäure, Katalysator und Ester in der Wärme zugegeben, Methylformiat abdestilliert und gleichmäßig Sumpfprodukt dem Reaktor entnommen und dieses Gemisch kontinuierlich oder diskontinuierlich aufgearbeitet

Das folgende Beispiel soll die Erfindung naher erlautern.

### Beispiel

Man benutzt eine Glasapparatur, die aus einem Vierhalskolben mit Rührer, Thermometer, Tropftrichter, Destillationskolonne mit aufgesetzter Destillationsbrucke und Vorlage besteht.

Man setzt ein:
- 750 g (7,5 mol): Cyclopropancarbonsäuremethylester (CPME)
- 521 g (11,3 mol): Ameisensäure
- 75 g: C₁₀- bis C₁₃-Alkylbenzolsulfonsäure (MARLON® AS 3-Säure)

Die Produkte werden zusammengegeben und auf über 105 °C erwärmt Das entstehende Methylformiat wird abdestilliert und dabei die Sumpftemperatur entsprechen erhöht, bis auf ca. 130 °C. Die Kopftemperatur liegt bei 38 °C. Nach 7,5 h ist die Umsetzung beendet Der CPME-Gehalt liegt bei 0,5 %

Das Reaktionsgemisch wird durch Abtoppen an einer Destillationsbrücke bei 133 h Pa vom Katalysator befreit. Es fallen 77 g Destillationsrückstand und 775 g Destillat an. Es entstehen also keine nennenswerten Mengen an nicht destillierbaren Hochsiedern. Der Rückstand wird als Katalysator wieder eingesetzt

Das abgetoppte Produktionsgemisch wird fraktioniert destilliert und 587 g Cyclopropancarbonsäure (CPC) mit einer Reinheit von 99,4 % erhalten. Die Ausbeute an CPC errechnet sich zu ca. 90 %, bezogen auf eingesetztes CPME.

### Vergleichsbeispiel

Man verwendet die im Beispiel 1 beschriebene Apparatur, setzt die dort genannten Mengen an CPME und Ameisensäure ein und gibt 30 g konz Schwefelsäure als Katalysator zu. Dieses Gemisch wird auf über 100 °C erwarmt. Ab 102 °C fängt die Destillation des Methylformiats an. Nach der Umsetzung wird abgekühlt und zur Neutralisation der Schwefelsaure zum Sumpfprodukt 20,4 g 50 %ige Natronlauge zugegeben. Dann wird direkt im Vakuum destilliert und CPC in einer Ausbeute von 87 % erhalten.

Bei dieser Destillation fallen 95 g eines teilweise festen Ruckstandes an, der in 150 ml Wasser gelöst wird. Diese Lösung enthält ca. 10 % CPC, und der Kohlenstoffgehalt liegt bei 7,5 %. Dieser Abfall kann in dieser Form nicht entsorgt werden, sondern muß erst durch Extraktionen oder Destillationen aufgearbeitet werden.

## Patentansprüche

1. Verfahren zur Herstellung von Cyclopropancarbonsäuren aus den entsprechenden Estern nach dem Schema 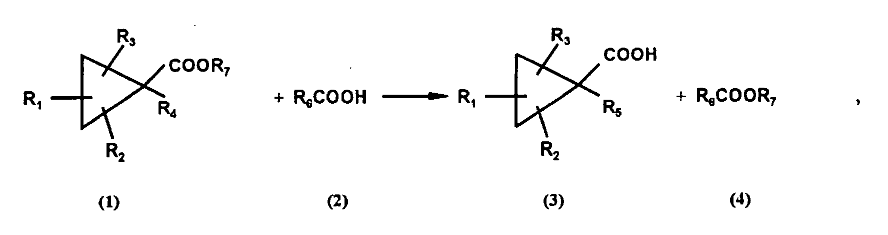 wobei
R₁, R₂, R₃ = H, C₁- bis C₄-Alkyl, Phenyl,
R₄ = H, COOR₇,
R₅ = H, COOR₇, COOH,
R₆ = H, CH₃, C₂H₅, C₃H₇,
R₇ = CH₃, C₂H₅ sein kann,
**dadurch gekennzeichnet,**
**daß** als Katalysator eine Alkylbenzolsulfonsäure der Struktur 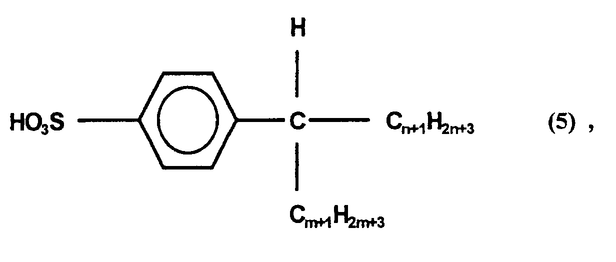 wobei m + n = 5 bis 15 ist, eingesetzt wird und wobei man das Reaktionsgemisch vom Katalysator abdestilliert.

2. Verfahren nach Anspruch 1,
**dadurch gekennzeichnet,**
**daß** m + n = 7 bis 10 ist.

3. Verfahren nach Anspruch 1,
**dadurch gekennzeichnet,**
**daß** man den Katalysator in Mengen von 5 bis 50 Gew.-%, bezogen auf den Cyclopropancarbonsäureester, einsetzt.

4. Verfahren nach Anspruch 1,
**dadurch gekennzeichnet,**
**daß** man die Reaktion bei 90 bis 150°C durchführt.

5. Verfahren nach Anspruch 1,
**dadurch gekennzeichnet,**
**daß** man das Reaktionsgemisch vom Katalysator abdestilliert und den Rückstand als Katalysator für einen neuen Ansatz verwendet.

## Claims

1. A method for preparing a cyclopropanecarboxylic acid from the corresponding ester according to the scheme 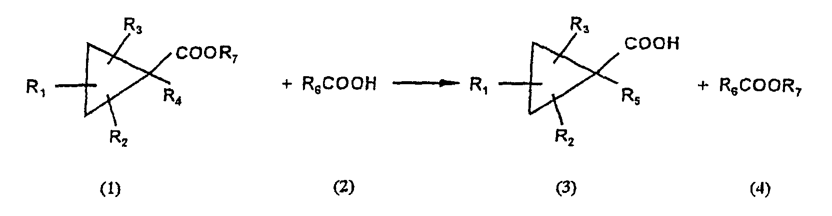 where the symbols have the following possible meanings
R₁, R₂, R₃ = H, C₁ - to C₄-alkyl, phenyl,
R₄ = H, COOR₇,
R₅ = H, COOR₇, COOH,
R₆ = H, CH₃, C₂H₅, C₃H₇,
R₇ = CH₃, C₂H₅,
**characterized in that**
the catalyst used is an alkylbenzenesulphonic acid having the structure 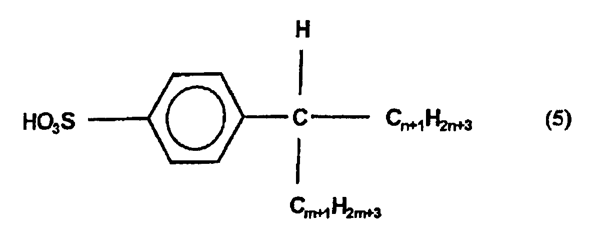 where m + n = 5 to 15, and the reaction mixture is distilled off from the catalyst

2. A method according to claim 1,
**characterized in that**
m + n = 7 to 10.

3. A method according to claim 1,
**characterized in that**
the catalyst is used in amounts of from 5 to 50wt%, based on the cyclopropanecarboxylic acid ester.

4. A method according to claim 1,
**characterized in that**
the reaction is carried out at from 90 to 150°C.

5. A method according to claim 1,
**characterized in that**
the reaction mixture is distilled off from the catalyst and the residue is used as a catalyst for a new batch.

## Revendications

1. Procédé de production d'acides cyclopropanecarboxyliques à partir des esters correspondants selon le schéma : 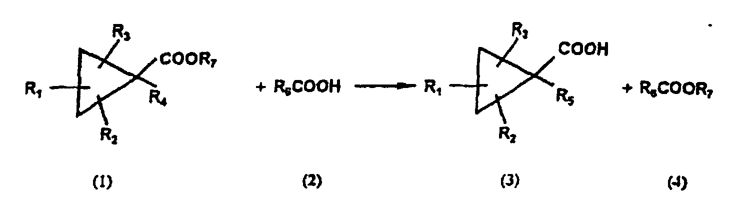 dans lequel on peut avoir
R₁, R₂, R₃ = H, Alkyle en C₁ à C₄, Phényle,
R₄ = H, COOR₇,
R₅ = H, COOR₇, COOH,
R₆ = H, CH₃, C₂H₅, C₃H₇,
R₇ = CH₃, C₂H₅
**caractérisé en ce que**
comme catalyseur on met en oeuvre un acide alkylbenzènesulfonique de structure 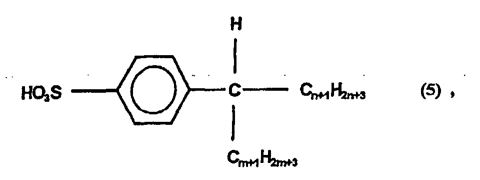 dans laquelle m + n = 5 à 15
et dans lequel on sépare le mélange réactionnel du catalyseur par distillation.

2. Procédé selon la revendication 1,
**caractérisé en ce que**
m + n = 7 à 10.

3. Procédé selon la revendication 1,
**caractérisé en ce qu'**
on met en oeuvre le catalyseur en quantité allant de 5 à 50 % en poids rapporté à l'ester d'acide cyclopropanecarboxylique.

4. Procédé selon la revendication 1,
**caractérisé en ce qu'**
on effectue la réaction de 90 à 150°C.

5. Procédé selon la revendication 1,
**caractérisé en ce qu'**
on sépare du catalyseur par distillation le mélange réactionnel et on utilise le résidu en tant que catalyseur, pour une nouvelle charge.
